# EUROPÄISCHE PATENTSCHRIFT

(11) **EP 3 444 329 B1**
(45) Veröffentlichungstag und Bekanntmachung des Hinweises auf die Patenterteilung: **12.02.2020**
(21) Anmeldenummer: 17186103.2
(22) Anmeldetag: 14.08.2017
(51) Int. Cl.: C12M 1/00, C12M 1/34, C12M 1/02, C12M 1/36

(54) **VORRICHTUNG ZUR ANFEUCHTUNG EINES GASGEMISCHES FÜR DIE ZELLINKUBATION**
DEVICE FOR THE HUMIDIFICATION OF A GAS MIXTURE FOR CELL INCUBATION
DISPOSITIF D'HUMIDIFICATION D'UN MÉLANGE GAZEUX PERMETTANT L'INCUBATION DE CELLULE

(43) Veröffentlichungstag der Anmeldung: 20.02.2019
(73) Patentinhaber: PeCon GmbH, 89155 Erbach (DE)
(72) Erfinder: Lawrinenko, Helmut, 88480 Achstetten OT Bronnen (DE)
(74) Vertreter: Baur & Weber Patentanwälte PartG mbB

(56) Entgegenhaltungen:
- EP-A1- 2 492 605
- EP-A1- 2 562 240
- EP-A1- 2 578 315
- EP-A1- 2 770 047
- EP-A2- 0 598 269
- DE-A1- 2 924 446
- DE-A1-102006 009 370
- DE-A1-102010 012 790
- DE-A1-102013 111 568
- DE-T5-112015 003 124

## Beschreibung

Die Erfindung betrifft eine Vorrichtung zur Anfeuchtung eines Gasgemisches für die Zellinkubation.

Eine Besonderheit von Inkubationssystemen für die Zellforschung besteht darin, dass diese Systeme direkt an Mikroskopen angebracht werden können, wodurch die zu beobachtenden Zellen am Mikroskop auch über längere Zeiträume unter physiologischen Bedingungen am Leben erhalten werden können. Dazu stellen derartige Inkubatoren, in denen sich Zellkulturgefäße mit Nährlösung und lebenden Zellen befinden, vorherbestimmte physikalische Bedingungen bereit. Neben einer geregelten Temperatur, die bei menschlichen Zellen typischerweise 37°C beträgt, bei tierischen Zellen jedoch auch größer oder kleiner sein kann, wird oftmals ein geregelter CO₂-Gehalt der durchströmten Luft eingestellt. Dieser beträgt typischerweise 5 % Vol., um einen definierten ph-Wert in der Nährlösung einzustellen, was üblicherweise in Abhängigkeit vom Natriumhydrogencarbonat-Gehalt der Nährlösung vorgenommen wird. Darüber hinaus müssen in manchen Anwendungen der Sauerstoffgehalt eingestellt werden, der sowohl dem der Umgebungsluft als auch dem im Inneren von Körpern von Tieren oder Menschen vorherrschenden Werten entsprechen kann. So ist beispielsweise der übliche Sauerstoffgehalt in der Umgebungsluft 20,8 % Vol., während in Körpern von Säugetieren der Sauerstoffgehalt im Bereich von 2 bis 10 % Vol. beträgt. Schließlich ist es auch vorgesehen, eine möglichst hohe relative Feuchte der inkubierten, aufbereiteten Luft bereitzustellen, so dass das Wasser in der Nährlösung nur eine geringe Verdunstungsrate aufweist und somit die lonenstärke der Nährlösung weitgehend konstant bleibt.

Aus dem allgemeinen Stand der Technik sind Befeuchtungseinrichtungen bekannt, die eine Regelung der oben beschriebenen physikalischen Bedingungen bewirken. So kann mittels einer Laborglasflasche, die mit destilliertem oder demineralisiertem Wasser befüllt ist, eine Luftanfeuchtung erreicht werden. Dazu wird über einen Schlauch mit einem Ausströmerstein die auf einen bestimmten CO₂-Gehalt aufbereitete Luft in Wasser eingeleitet, welches beheizt wird. Die an der Wasseroberfläche austretenden Luftblasen reißen Wassermoleküle mit sich, so dass die Luft oberhalb des Wassers in der Laborglasflasche stark angefeuchtet ist. Die angefeuchtete Luft wird über einen Schlauch an den Inkubator geleitet, der von ihr mit geringem Volumenstrom durchflutet wird.

Ein derartiges System weist jedoch physikalische Grenzen auf, die insbesondere darin bestehen, dass die im Inkubationsraum erzielbare relative Feuchte von der Wärme des durchfluteten Wassers abhängt. Diese lässt sich jedoch wegen der hohen Wärmekapazität des Wassers und des schlechten Wärmeübergangs zwischen einer Heizeinrichtung und der Laborglasflasche nur sehr langsam und ungenau steuern. Weitere Nachteile bestehen beispielsweise darin, dass ein Schlauch, in welchem die befeuchtete Luft von der Laborglasflasche zum Inkubationsraum transportiert wird, eine geringere Temperatur als der Inkubationsraum aufweist, wobei beim Arbeiten mit hoher relativer Feuchte Wasser im Schlauch kondensiert, so dass in den Inkubationsraum Wassertropfen geblasen werden, was schädliche Auswirkungen auf die technischen Systeme im Inkubator oder die dort befindlichen Zellkulturen haben könnte, oder durch Niederschlagen der Tropfen auf das Deckglas das von oben einfallende Licht inhomogen zerstreuen könnte. Darüber hinaus wurde beobachtet, dass die Erwärmung des Wassers in der Flasche über einen längeren Zeitraum zu einer Verkeimung des Systems führen kann. Da dem Wasser keine keimtötenden Mittel zugesetzt werden dürfen, da diese in die feuchte Luft verschleppt und dann die Zellkulturen schädigen könnten, müssen daher das Wasser und der Ausströmerstein in der Laborglasflasche häufig getauscht, oder zumindest gereinigt und sterilisiert werden.

Desweiteren ist aus der EP 2 770 047 A1 eine Vorrichtung zum Bereitstellen eines Gases zum Einleiten in eine Inkubationskammer bekannt, wobei die Vorrichtung einen Einlassanschluss zum Einleiten von einem Gas, einen Auslassanschluss zum Ausleiten von einem Gas, einen ersten Gasweg, der den Einlassanschluss mit dem Auslassanschluss verbindet, einen zweiten Gasweg, der vom ersten Gasweg abzweigt und wieder in den ersten Gasweg mündet, ein Ventilelement, das so ausgebildet ist, dass sein Anteil des durch den Einlassanschluss eingeleiteten Gases über den zweiten Gasweg leitbar ist, und ein im zweiten Gasweg angeordnetes Anreicherungselement zum Anreichern des durchströmten Gases mit einer Flüssigkeit umfasst.

Aus der EP 2 492 605 A1 ist ein Verfahren zum Regeln der Temperatur oder Feuchtigkeit eines Gases bekannt. Dabei wird basierend auf einer gewünschten Temperatur und einer gewünschten relativen Feuchtigkeit eine Zwischentemperatur berechnet. Eingebrachtes Gas wird mit Wasserdampf vermischt, um die Temperatur des Gases auf die Zwischentemperatur zu erhöhen und die relative Feuchtigkeit des Gases auf 100 % zu erhöhen. Daraufhin wird das Gas auf die gewünschte Temperatur erhitzt, während die Wasserdampfmenge beibehalten wird, so dass die relative Feuchtigkeit auf den gewünschten Wert eingestellt wird.

Aus der EP 2562240 A1 ist Laboratoriums-Brutschrank, insbesondere Begasungs-Brutschrank, mit einem von einem Gehäuse umgebenen Innenraum und einem außerhalb des Innenraums angeordneten Dampferzeuger, der mit dem Innenraum über eine Dampfzuleitung in Verbindung steht, bekannt. Der Dampferzeuger ist als im Wesentlichen druckloser Behälter mit einem Bodenbereich und einem darüberliegenden Dampfraum ausgebildet, wobei der Bodenbereich zur Aufnahme eines Wasserreservoirs ausgebildet und mit einer Heizvorrichtung zur Erwärmung des Wasserreservoirs versehen ist, die Dampfzuleitung den Dampferzeuger im Bereich des Dampfraumes verlässt und eine Luftzuleitung in den Dampferzeuger einmündet, um dem Dampferzeuger Umgebungsluft zuzuführen und mit Wasserdampf angereicherte Luft über die Dampfzuleitung im Wesentlichen drucklos in den Innenraum des Laboratoriums-Brutschranks einzuleiten.

Aus der DE 10 2010 012 790 A1 ist eine Inkubatoreinrichtung mit einer Kulturkammer zur Aufnahme in einer Zellkultur, einer Temperierungseinrichtung zum Einstellen der Temperatur in der Kulturkammer, einer Gaszufuhreinrichtung zum Zuführen von Gas in die Kulturkammer, und mit einer Energiespeichereinrichtung bekannt, die zur Versorgung der Temperierungseinrichtung und/oder der Gaszufuhreinrichtung mit elektrischer Energie angeordnet ist. Desweiteren weist die Inkubatoreinrichtung ein Datenaufzeichnungssystem auf, mit dem die Temperatur und/oder die Gaszusammensetzung in der Kulturkammer und/oder in der Umgebung der Inkubatoreinrichtung elektronisch speicherbar sind.

Zwar ermöglicht die in der ersten Schrift beschriebene Lösung eine schnelle Regelbarkeit der Feuchte, wobei aber das Wasser zunächst einige Zeit vorgeheizt werden muss, löst jedoch den oben beschriebenen Nachteil der möglichen Verkeimung nicht.

Ausgehend von diesem Stand der Technik hat sich der Erfinder nun die Aufgabe gestellt, eine Vorrichtung zu schaffen, die eine verbesserte Anfeuchtung eines Gasgemisches für die Zellinkubation ermöglicht.

Diese Aufgabe wird durch die Merkmale des Patentanspruchs 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird eine Vorrichtung zur Anfeuchtung eines Gasgemisches für die Zellinkubation angegeben. Die Vorrichtung weist ein Reservoir für Wasser, einen Verdampfer, einen Gaseinlass zur Einleitung des Gasgemisches an den Verdampfer, dem Wasser aus dem Reservoir zugeführt wird, und eine beheizbare Messkammer mit einem Temperatursensor und einem Feuchtesensor auf. Der Messkammer ist das mit Wasser angereicherte Gasgemisch aus dem Verdampfer zugeführt, so dass das mit Wasser angereicherte Gasgemisch nach Weiterleitung in einen Inkubationsraum auf einen vorgegebenen Sollwert bezüglich der relativen Feuchte bei einer vorgegebenen Temperatur durch Dosierung der Wasserzufuhr am Verdampfer geregelt ist, wobei die Temperatur in der Messkammer höher als die Temperatur im Inkubationsraum ist.

Demnach wird bei der erfindungsgemäßen Vorrichtung eine Anfeuchtung des Gasgemisches im Inkubationsraum dadurch erreicht, dass das Gasgemisch mit Wasser aus dem Verdampfer über die Messkammer in den Inkubationsraum weitergeleitet wird, wobei zur Regelung Sensoren zur Bestimmung der Temperatur in der Messkammer sowie der relativen Feuchte zur Verfügung stehen. Während im Stand der Technik die relative Feuchte im Inkubationsraum gemessen und geregelt wird, verfügt die erfindungsgemäße Vorrichtung über eine Regelung, so dass sich im Inkubationsraum die gewünschte relative Feuchte einstellt. Dies ist möglich, weil im gesamten Luftpfad die absolute Wassermasse pro Luftmasse bzw. bei eingeregelten Temperaturen pro Luftvolumen gleich bleibt. Da der Feuchtesensor in der Messkammer und nicht im weit abgesetzten Inkubationsraum angeordnet ist, wird eine lange Totzeit vermieden, die regelungstechnisch schwer zu beherrschen wäre. Die Anordnung des Feuchtesensors in der Messkammer führt daher zu einer schnelleren und stabileren Regelung. Um die relative Feuchte zu erhöhen oder zu verringern, wird die Zuführung von Wasser aus dem Reservoir dosiert. Während im Stand der Technik zuerst ein großes Wasserreservoir erwärmt oder abgekühlt werden muss, kann erfindungsgemäß eine wesentlich schnellere Regelung erfolgen.

Gemäß einer Ausführungsform der Erfindung erfolgt die Weiterleitung in den Inkubationsraum mittels eines beheizten Schlauches. Dabei kann die Temperatur im beheizten Schlauch höher als die Temperatur im Inkubationsraum sein. Bevorzugt ist der Schlauch elektrisch beheizbar.

Aufgrund dieser Vorgehensweise kann eine Kondensation von Wasser im Schlauch vermieden werden, wobei die Anbindung einer Befeuchtungseinrichtung an den Inkubationsraum mittels eines Schlauches an sich bereits aus dem Stand der Technik bekannt ist. Die erfindungsgemäße Vorrichtung ermöglicht anhand der bekannten Zieltemperatur im Inkubationsraum sowie der tatsächlichen Temperatur in der Messkammer das Beheizen des Schlauches auch automatisch zu regeln.

Gemäß einer weiteren Ausführungsform der Erfindung ist der Verdampfer mit einer Temperatur betreibbar, die so gewählt ist, dass eine Verkeimung des mit Wasser angereicherten Gasgemisches verringert ist.

Typischerweise wird die möglicherweise verkeimte Raumluft im Verdampfer auf 135°C erhitzt, so dass diese weitestgehend keimfrei ist. Eine derartige Vorgehensweise ist bei den Befeuchtungseinrichtungen nach dem Stand der Technik nicht möglich gewesen, da derartig hohe Temperaturen aufgrund der gewählten Gerätekonfiguration nicht erreicht werden mussten. Das Bereitstellen einer keimfreien Umgebung ist besonders vorteilhaft, da folglich auch sämtliche bisher durchzuführende Wartungsarbeiten entfallen können.

Gemäß einer weiteren Ausführungsform der Erfindung ist das Gasgemisch vor Einleitung in den Verdampfer einem ersten Anschluss an einem ersten Kreis eines Wärmetauschers zugeführt werden, wobei der zweite Anschluss am ersten Kreis des Wärmetauschers mit dem Eingang des einen Mäander aufweisenden Verdampfers verbunden ist. Dabei kann der erste Anschluss an einem zweiten Kreis des Wärmetauschers mit dem Ausgang des Verdampfers verbunden sein. Der Wärmetauscher kann Bestandteil der Messkammer sein, wobei bevorzugt der zweite Anschluss am zweiten Kreis des Wärmetauschers einen Auslass der Messkammer bildet.

Das Gasgemisch, typischerweise mit CO₂ angereicherte Luft, wird demnach zuerst durch die Messkammer geleitet, wobei sie dieser Wärmeenergie entzieht. Das somit vorgewärmte Gasgemisch wird anschließend durch eine Mäanderstruktur des Verdampfers geleitet, der, wie oben erwähnt, typischerweise kontrolliert auf 135°C oder höher erhitzt wird. In den Verdampfer wird zusätzlich kontrolliert Wasser in kleinen Dosen gepumpt, welches verdampft und sich somit mit dem Luft-CO₂-Gasstrom als Gasgemisch vermischt. Dieses mit Wasser angereicherte Gasgemisch durchströmt wiederum die Messkammer. Dazu wird der zweite Zweig des Wärmetauschers parallel zum ersten Zweig des einströmenden Gasgemisches geführt. Der erste Zweig und der zweite Zweig bilden somit einen Wärmetauscher. Während das Luft-CO₂-Wasserdampfgemisch viel Wärmeenergie beinhaltet, welche in die Messkammer eingetragen wird, wird ein Teil der Wärmeenergie über das ankommende Gasgemisch, das Raumtemperatur hat, und die über die parallel verlaufenden Kanäle geführt wird, wieder entnommen. Diese Wärmeenergie ist jedoch nicht verloren, da das nun vorgewärmte Gasgemisch somit im Verdampfer weniger Heizenergie benötigt, um auf beispielsweise 135°C erwärmt zu werden. Durch diese Maßnahme verringert sich die Temperatur in der Messkammer deutlich. Im Gegenzug wird die Messkammer aktiv auf eine definierte Temperatur beheizt, welche etwas höher ist, als die Temperatur die sich ohne Beheizung einstellen würde. Durch diese Maßnahme wird eine definierte Temperatur in der Messkammer erzielt.

Gemäß einer weiteren Ausführungsform der Erfindung sind der Temperatursensor und der Feuchtesensor als Kombinationssensor an einem Flansch am Auslass der Messkammer angeordnet.

Diese Vorgehensweise ermöglicht einen kompakten Aufbau am Auslass der Messkammer, wobei insbesondere sichergestellt ist, dass das mit Wasser angereicherte Gasgemisch durch den Kombinationssensor beim Verlassen der Messkammer bezüglich der Parametertemperatur und -feuchte untersucht wird.

Gemäß einer weiteren Ausführungsform der Erfindung ist eine Regelschaltung vorgesehen, die mit dem Temperatursensor und dem Feuchtesensor zur Erfassung von Ist-Werten über erste und zweite Datenleitungen verbunden ist. Der Regelschaltung kann ein Ist-Wert der Temperatur im Inkubationsraum zugeführt werden, das vorzugsweise über eine dritte Datenleitung erfolgt. Dabei kann der Soll-Wert der relativen Feuchte an einer Eingabeeinheit eingebbar und an die Regelschaltung über eine vierte Datenleitung übertragbar sein. Die Regelschaltung kann aus den Ist-Werten eine absolute Wassermasse errechnen, aus der ein Ist-Wert einer relativen Feuchte im Inkubationsraum abgeleitet werden kann.

Um die relative Feuchte im Inkubationsraum einstellen zu können, wird eine Regelschaltung verwendet, deren Eingangsgrößen zum einen die Messwerte des Feuchtesensors und des Temperatursensors bilden. Zum anderen muss jedoch noch der gewünschte Feuchtesollwert sowie die aktuelle Temperatur im Inkubatorraum an die Regelschaltung übermittelt werden, um die absolute zu dosierende Wassermenge zu errechnen. Der gewünschte Feuchtesollwert kann auf einfache Weise über die Eingabeeinheit eingegeben werden, wobei dieses sowohl mit der Regelschaltung gemeinsam aber auch getrennt angeordnet sein kann. Unter Datenleitung wird selbstverständlich auch eine Übermittlung mittels Funksignalen verstanden. Da häufig im Inkubationsraum bereits eine eigene Steuereinrichtung für die gewünschte Temperatur in Form eines fremden Regelgeräts vorhanden ist, müssen geeignete Maßnahmen getroffen werden, um diesen den Inkubationsraum betreffenden Wert ebenfalls an die Regelschaltung weiterzugeben. Dies könnte beispielsweise dadurch erfolgen, dass das nicht Vorliegen von übermittelten Temperaturwerten im Inkubationsraum durch die Regelschaltung automatisch erkannt wird, so dass der Benutzer oder die Benutzerin aufgefordert werden, die fehlenden Werte beispielsweise von Hand über die Eingabeeinheit einzugeben.

Gemäß einer weiteren Ausführungsform der Erfindung ist das Wasserreservoir zur Regelung mit einer dosierbaren Mikropumpe versehen.

Auf diese Weise kann die Dosierung der Wasserzufuhr einfach und zuverlässig erfolgen, so dass eine kostengünstige Realisierung der erfindungsgemäßen Vorrichtung möglich ist.

Gemäß einer weiteren Ausführungsform der Erfindung sind der Schlauch und/oder das Reservoir so ausgeführt, dass diese jeweils sterilisierbar sind.

Neben der bereits erwähnten Keimfreiheit stellt die erfindungsgemäße Vorrichtung auch die Möglichkeit zur Sterilisierung der Schlauchs bzw. des Reservoirs zur Verfügung, was durch geeignete Materialauswahl, die beispielsweise so gewählt werden müssen, dass eine bestimmte zur Sterilisierung notwendige Temperatur ohne Degradation verwendet werden kann. Als geeignete Sterilisierungsmethoden kommen dabei sowohl Trockensterilisierung beispielsweise bei einer Temperatur von 165°C beim Schlauch sowie zusätzlich eine Autoklavierbarkeit beim Reservoir in Frage. Die weiteren Komponenten der erfindungsgemäßen Vorrichtung bleiben, ohnehin wegen des Verdampfers von sich aus weitestgehend steril, wie oben beschrieben.

Nachfolgend werden einige Ausführungsbeispiele anhand der Zeichnung näher erläutert. Es zeigt:

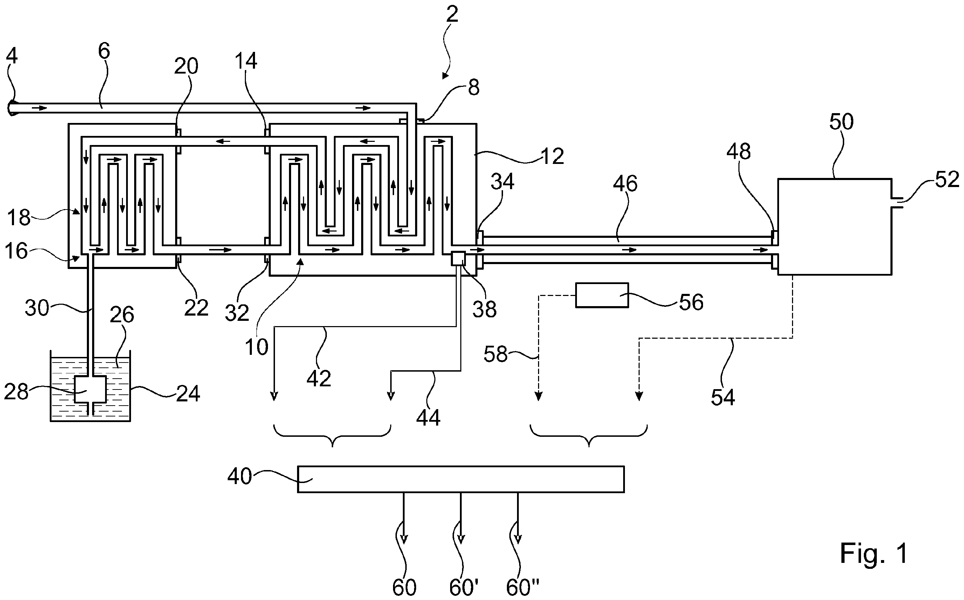

- Fig. 1: eine erfindungsgemäße Vorrichtung in einer schematischen Ansicht.

In den Figuren sind gleiche oder funktional gleich wirkende Bauteile mit den gleichen Bezugszeichen versehen.

In Fig. 1 wird nachfolgend eine erste Ausführungsform einer Vorrichtung 2 zur Anfeuchtung eines Gasgemisches für die Zellinkubation gezeigt. Die Vorrichtung 2 weist einen Gaseinlass 4 auf, über den ein Gasgemisch eingeleitet werden kann. Hierbei handelt es sich typischerweise um mit Kohlendioxid angereicherte Luft von einem Gasregelgerät. Die Strömungsrichtung des Gasgemisches im Inneren einer sich an den Gaseinlass 4 anschließenden Röhre 6 ist in Fig. 1 mittels kurzer Pfeile ohne Bezugszeichen dargestellt. Es versteht sich von selbst, dass die gezeigte Röhre 6 auch durch eine Vielzahl einzelner miteinander verbindbarer Elemente gebildet werden kann.

Das Gasgemisch wird über einen ersten Anschluss 8 einem ersten Zweig eines Wärmetauschers 10 zugeführt, der im Inneren einer Messkammer 12 angeordnet ist. Das Gasgemisch verlässt den ersten Zweig des Wärmetauschers 10 über den zweiten Anschluss 14 und wird einem Verdampfer 16 zugeführt. Der Verdampfer 16 weist im Inneren eine Mäanderstruktur 18 auf, die zwischen dem Eingang 20 und dem Ausgang 22 des Verdampfers 16 gebildet ist.

Desweiteren ist ein Reservoir 24 vorgesehen, das destilliertes oder demineralisiertes Wasser 26 über eine Mikropumpe 28 und einer Zuführungsleitung 30 kontrolliert der Mäanderstruktur 18 des Verdampfers 16 zuführt. Der Verdampfer 16 wird in seinem Inneren auf ca. 135°C erhitzt, so dass das aus dem Reservoir 24 stammende Wasser 26 verdampft, so dass am Ausgang 22 des Verdampfers 16 das Gasgemisch nun in Form eines mit Wasserdampf angereicherten Gasgemisches vorliegt.

Das mit Wasserdampf angereicherte Gasgemisch wird über einen ersten Anschluss 32 eines zweiten Zweigs des Wärmetauschers 10 wieder in die Messkammer 12 zurückgeführt und verläuft dort im Wesentlichen parallel zum ersten Zweig des Verdampfers 10. Der zweite Anschluss des zweiten Zweigs des Verdampfers 10 wird durch einen Flansch am Auslass 34 der Messkammer 12 gebildet.

Am Flansch als Auslass 34 der Messkammer 12 ist ein Kombinationssensor 38 angeordnet, der von dem ausströmenden mit Wasserdampf angereicherten Gasgemisch durchströmt wird. Der Kombinationssensor 38 umfasst einen Temperatursensor und einen Feuchtesensor zur Bestimmung der relativen Feuchte des mit Wasserdampf angereicherten Gasgemisches. Die Messsignale bezüglich Temperatur und Feuchte werden aus der Messkammer 12 an eine Regelschaltung 40 weitergegeben, wie schematisch mittels einer ersten Datenleitung 42 und einer zweiten Datenleitung 44 angedeutet ist. Der Begriff "Datenleitung" umfasst hier sowohl die kabel- als auch die kabellose Übertragung. Es versteht sich von selbst, dass die Regelschaltung 40 in anderen Ausführungsformen auch Bestandteil der Messkammer 12 oder eines anderen Teils der Vorrichtung 2 sein kann.

Am Flansch als Auslass 34 befindet sich ein beheizbarer Schlauch 46, der über ein geeignetes Anschlussstück 48 mit einem beispielsweise ein Zellkulturgefäß beinhaltenden Inkubationsraum 50 verbunden ist. Somit gelangt über den Schlauch 46 das mit Wasserdampf angereicherte Gasgemisch in das Innere des Inkubationsraums 50, der wie schematisch angedeutet, eine Leckageöffnung 52 umfasst.

Desweiteren wird eine im Inneren des Inkubationsraums 50 gemessene Temperatur an die Regelschaltung 40 weitergegeben, wie anhand der dritten Datenleitung 54 in Fig. 1 angedeutet ist. Da die Temperatur im Inneren des Inkubationsraums 50 typischerweise auf einen festen Wert wie z. B. 37°C geregelt wird, kann dieser Wert auch von Hand durch eine Benutzerin oder einen Benutzer eingegeben werden.

Als weitere Größe wird der Sollwert der Feuchte im Inneren des Inkubationsraums über eine Eingabeeinheit 56 durch eine Benutzerin oder einem Benutzer festgelegt, wobei dieser Wert ebenfalls an die Regelschaltung 40 weitergeleitet wird, wie schematisch anhand der vierten Datenleitung 58 angedeutet ist. Die Eingabeeinheit 56 kann auch dafür verwendet werden, den Temperaturwert im Inneren des Inkubationsraums 50 wie oben beschrieben von Hand einzugeben. Als Ausgangssignale stellt die Regelschaltung mehrere in Figur 1 lediglich beispielhaft und schematisch mit den Bezugszeichen 60, 60' und 60" versehene Ausgänge bereit, die an unterschiedlichen Stellen eine Regelung der Feuchte mittels der Vorrichtung 2 bewirken. Die detaillierte Vorgehensweise bezüglich der Regelung wird nachfolgend noch genauer erläutert werden.

Zur Regelung der relativen Feuchte im Inkubationsraum 50 wird die Zuführung von Wasser 26 aus dem Reservoir 24 in den Verdampfer 16 dosiert. Die mittels des Kombinationssensors 38 gemessenen Werte der Temperatur und der relativen Feuchte in der Messkammer 12 werden verwendet, um eine absolute Wassermasse als Ist-Wert zu berechnen. Anhand der Temperatur im Inkubationsraum 50 sowie dem Sollwert der relativen Feuchte im Inkubationsraum 50 wird eine absolute Wassermasse im Inkubationsraum als Soll-Wert berechnet. Durch einen Vergleich des Ist-Werts mit dem Soll-Wert wird eine Regelung der absoluten Wassermasse durchgeführt. Über die Regelung der absoluten Wassermasse wird daher erreicht, dass sich im Inkubationsraum 50 die gewünschte relative Feuchte einstellt.

Es sei an dieser Stelle erwähnt, dass der Druck des Gasgemisches nur einen geringen Einfluss auf die absolute Feuchte ausübt, da sich beispielsweise bei einer Druckerhöhung auch die Partialdrücke der verschiedenen Gase im Gasgemisch proportional erhöhen, so dass das Verhältnis gleich bleibt. Erst wenn sich aufgrund der Druckänderung eine Volumenänderung ergeben würde, würde sich das auf die absolute Feuchte auswirken. Da jedoch die Vorrichtung 2 pneumatisch betrachtet "offen" ist, bleibt im gesamten Pfad vom Gaseintritt am Gaseinlass 4 bis zum Austritt des Gasgemisches an der Leckageöffnung 52 das Systemvolumen und damit auch der Volumenstrom konstant.

Die Regelschaltung 40 benötigt den Ist-Wert der relativen Feuchte im Inkubationsraum 50 aufgrund der Regelung der Wassermasse zwar nicht, dieser muss jedoch für eine Benutzerin oder einen Benutzer auslesbar als Information zur Verfügung stehen. Dieser Wert lässt sich jedoch auf einfache Weise aus dem Ist-Wert der absoluten Masse errechnen.

Die erfindungsgemäße Vorrichtung 2 weist zahlreiche Vorteile gegenüber den bisher bekannten Befeuchtungseinrichtungen auf. So sitzt der Feuchtesensor nicht im weit abgesetzten Inkubationsraum, was eine lange Totzeit vermeidet, die regelungstechnisch schwer zu beherrschen wäre. Dadurch wird eine stabile und schnelle Regelung erreicht. Desweiteren muss kein großes Wasserreservoir erwärmt oder abgekühlt werden, um die relative Feuchte im Inkubationsraum 50 zu erhöhen oder zu verringern. Auch dies führt zu einer schnellen Regelung. Da die verkeimte Raumluft im Verdampfer beispielsweise auf 135°C erhitzt wird, ist diese weitestgehend keimfrei. Durch das Ausbilden der Messkammer 12 mit einem Wärmetauscher 10 benötigt der Verdampfer 16 weniger Heizenergie, da über den Wärmetauscher 10 das Gasgemisch vorgewärmt wird. Dies führt auch zu einer definierten Temperatur im Inneren der Messkammer. Das mit Wasserdampf angereicherte Gasgemisch durchströmt dann den beheizten Schlauch 46, dessen Temperatur etwas höher eingestellt wird als die Temperatur im Inkubationsraum 50, so dass sichergestellt ist, dass im Schlauch 46 kein Wasser kondensiert.

Die erfindungsgemäße Vorrichtung 2 kann sowohl als Einzelteile als auch in Form eines Gesamtsystems bereitgestellt werden. Typischerweise wäre jedoch der Inkubationsraum 50 nicht Bestandteil eines derartigen Gesamtsystems. Des Weiteren wäre der Schlauch 46 üblicherweise abnehmbar. Die weiteren Komponenten können zu einer Einheit verbaut sein.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

### Liste der Bezugszeichen:

- 2: Vorrichtung
- 4: Gaseinlass
- 6: Röhre
- 8: erster Anschluss
- 10: Wärmetauscher
- 12: Messkammer
- 14: zweiter Anschluss
- 16: Verdampfer
- 18: Mäanderstruktur
- 20: Eingang
- 22: Ausgang
- 24: Reservoir
- 26: Wasser
- 28: Mikropumpe
- 30: Zuführungsleitung
- 32: erster Anschluss
- 34: Auslass
- 38: Kombinationssensor
- 40: Regelschaltung
- 42, 44: erste, zweite Datenleitung
- 46: Schlauch
- 48: Anschlussstück
- 50: Inkubationsraum
- 52: Leckageöffnung
- 54: dritte Datenleitung
- 56: Eingabeeinheit
- 58: vierte Datenleitung

## Patentansprüche

1. Vorrichtung zur Anfeuchtung eines Gasgemisches für die Zellinkubation aufweisend
ein Reservoir (24) für Wasser,
einen Verdampfer (16),
einen Gaseinlass (4) zur Einleitung des Gasgemisches an den Verdampfer (16), dem Wasser (26) aus dem Reservoir (24) zugeführt ist,
und eine beheizbare Messkammer (12) mit einem Temperatursensor und einem Feuchtesensor, der das mit Wasser angereicherte Gasgemisch aus dem Verdampfer (16) zugeführt ist, so dass das mit Wasser angereicherte Gasgemisch nach Weiterleitung in einen Inkubationsraum (50) auf einen vorgegebenen Sollwert bezüglich der relativen Feuchte bei einer vorgegebenen Temperatur durch Dosierung der Wasserzufuhr am Verdampfer (16) geregelt ist, wobei die Temperatur in der Messkammer (12) höher als die Temperatur im Inkubationsraum (50) ist.

2. Vorrichtung nach Anspruch 1, bei der die Weiterleitung in den Inkubationsraum (50) mittels eines beheizten Schlauches (46) erfolgt.

3. Vorrichtung nach Anspruch 2, bei der die Temperatur im beheizten Schlauch (46) höher als die Temperatur im Inkubationsraum (50) ist.

4. Vorrichtung nach Anspruch 2 oder 3, bei der der Schlauch (46) elektrisch beheizbar ist.

5. Vorrichtung nach einem der Ansprüche 1 bis 4, bei der der Verdampfer (16) mit einer Temperatur betreibbar ist, die so gewählt ist, dass eine Verkeimung des mit Wasser angereicherten Gasgemisches verringert ist.

6. Vorrichtung nach einem der Ansprüche 1 bis 5, bei der das Gasgemisch vor Einleitung in den Verdampfer (16) einem ersten Anschluss (8) an einem ersten Kreis eines Wärmetauschers (10) zuführbar ist, wobei der zweite Anschluss (14) am ersten Kreis des Wärmetauschers (10) mit dem Eingang (20) des eine Mäanderstruktur (18) aufweisenden Verdampfers (16) verbunden ist.

7. Vorrichtung nach Anspruch 6, bei der der erste Anschluss (32) an einem zweiten Kreis des Wärmetauschers (10) mit dem Ausgang (22) des Verdampfers (16) verbunden ist.

8. Vorrichtung nach Anspruch 6 oder 7, bei der der Wärmetauscher (10) Bestandteil der Messkammer (12) ist, wobei vorzugsweise der zweite Anschluss am zweiten Kreis des Wärmetauschers (10) einen Auslass (34) der Messkammer (12) bildet.

9. Vorrichtung nach Anspruch 8, bei der der Temperatursensor und der Feuchtesensor als Kombinationssensor (38) an einem Flansch am Auslass (34) der Messkammer (12) angeordnet sind.

10. Vorrichtung nach einem der Ansprüche 1 bis 9, bei der eine Regelschaltung (40) vorgesehen ist, die mit dem Temperatursensor und dem Feuchtesensor zur Erfassung von Ist-Werten über erste und zweite Datenleitungen (42; 44) verbunden ist.

11. Vorrichtung nach einem der Ansprüche 10, bei der der Regelschaltung (40) ein Ist-Wert der Temperatur im Inkubationsraum (50) zuführbar ist, vorzugsweise über eine dritte Datenleitung (54).

12. Vorrichtung nach Anspruch 10, bei der der Soll-Wert der relativen Feuchte an einer Eingabeeinheit (56) eingebbar und an die Regelschaltung (40) über eine vierte Datenleitung (58) übertragbar ist.

13. Vorrichtung nach einem der Ansprüche 11 oder 12, bei der die Regelschaltung (40) aus den Ist-Werten eine absolute Wassermasse errechnet, aus der ein Ist-Wert einer relativen Feuchte im Inkubationsraum (50) ableitbar ist.

14. Vorrichtung nach einem der Ansprüche 1 bis 13, bei der zur Regelung das Wasserreservoir (24) mit einer dosierbaren Mikropumpe (28) versehen ist.

15. Vorrichtung nach einem der Ansprüche 1 bis 14, bei der der Schlauch 46) und/oder das Reservoir (24) so ausgeführt sind, dass diese jeweils sterilisierbar sind.

## Claims

1. Device for humidifying a gas mixture for cell incubation, comprising a reservoir (24) for water, an evaporator (16), a gas inlet (4) for introducing the gas mixture into the evaporator (16), to which water (26) from the reservoir (24) is supplied, and a heatable measuring chamber (12) having a temperature sensor and a humidity sensor, to which chamber the water-enriched gas mixture is supplied from the evaporator (16), such that the water-enriched gas mixture, after being further conducted into an incubation chamber (50), is controlled to a predefined target value with respect to the relative humidity at a predefined temperature by metering the water supply to the evaporator (16), wherein the temperature in the measuring chamber (12) is higher than the temperature in the incubation chamber (50).

2. Device according to claim 1, wherein the further conduction into the incubation chamber (50) takes place by means of a heated tube (46).

3. Device according to claim 2, wherein the temperature in the heated tube (46) is higher than the temperature in the incubation chamber (50).

4. Device according to either claim 2 or claim 3, wherein the tube (46) can be electrically heated.

5. Device according to any of claims 1 to 4, wherein the evaporator (16) can be operated at a temperature which is selected such that a bacterial contamination of the water-enriched gas mixture is reduced.

6. Device according to any of claims 1 to 5, wherein, before being introduced into the evaporator (16), the gas mixture can be supplied to a first port (8) on a first circuit of a heat exchanger (10), the second port (14) on the first circuit of the heat exchanger (10) being connected to the input (20) of the evaporator (16) having a meander structure (18).

7. Device according to claim 6, wherein the first port (32) on a second circuit of the heat exchanger (10) is connected to the output (22) of the evaporator (16).

8. Device according to either claim 6 or claim 7, wherein the heat exchanger (10) is a component of the measuring chamber (12), with preferably the second port on the second circuit of the heat exchanger (10) forming an outlet (34) of the measuring chamber (12).

9. Device according to claim 8, wherein the temperature sensor and the humidity sensor are arranged as a combination sensor (38) on a flange at the outlet (34) of the measuring chamber (12).

10. Device according to any of claims 1 to 9, wherein a control circuit (40) is provided which is connected to the temperature sensor and the humidity sensor for detecting actual values via first and second data lines (42; 44).

11. Device according to any of claims 10, wherein an actual value of the temperature in the incubation chamber (50) can be supplied to the control circuit (40), preferably via a third data line (54).

12. Device according to claim 10, wherein the target value of the relative humidity can be entered at an input unit (56) and can be transferred to the control circuit (40) via a fourth data line (58).

13. Device according to either claim 11 or claim 12, wherein the control circuit (40) calculates an absolute water mass from the actual values, from which mass an actual value of a relative humidity in the incubation chamber (50) can be derived.

14. Device according to any of claims 1 to 13, wherein the water reservoir (24) is provided with a metered micro-pump (28) for control.

15. Device according to any of claims 1 to 14, wherein the tube (46) and/or the reservoir (24) are designed such that they can each be sterilized.

## Revendications

1. Dispositif d'humidification d'un mélange gazeux permettant l'incubation de cellule, comprenant un réservoir (24) d'eau, un évaporateur (16), une entrée de gaz (4) permettant d'introduire le mélange gazeux dans l'évaporateur (16), auquel de l'eau (26) provenant du réservoir (24) est fournie, et une chambre de mesure (12) pouvant être chauffée, comportant un capteur de température et un capteur d'humidité alimenté par le mélange gazeux enrichi en eau provenant de l'évaporateur (16), de sorte que le mélange gazeux enrichi en eau, après avoir été acheminé dans une chambre d'incubation (50), soit réglé sur une valeur de consigne prédéfinie par rapport à l'humidité relative à une température prédéfinie par le dosage de l'alimentation en eau au niveau de l'évaporateur (16), la température dans la chambre de mesure (12) étant supérieure à la température dans la chambre d'incubation (50).

2. Dispositif selon la revendication 1, dans lequel l'acheminement dans la chambre d'incubation (50) s'effectue au moyen d'un tuyau (46) chauffé.

3. Dispositif selon la revendication 2, dans lequel la température dans le tuyau (46) chauffé est supérieure à la température dans la chambre d'incubation (50).

4. Dispositif selon la revendication 2 ou 3, dans lequel le tuyau (46) peut être chauffé électriquement.

5. Dispositif selon l'une des revendications 1 à 4, dans lequel l'évaporateur (16) peut fonctionner à une température qui est sélectionnée de manière à réduire la contamination du mélange gazeux enrichi en eau.

6. Dispositif selon l'une des revendications 1 à 5, dans lequel le mélange gazeux peut être acheminé, avant son introduction dans l'évaporateur (16), vers un premier port (8) au niveau d'un premier circuit d'un échangeur de chaleur (10), le second port (14) au niveau du premier circuit de l'échangeur de chaleur (10) étant relié à l'entrée (20) de l'évaporateur (16) comportant une structure sinueuse (18).

7. Dispositif selon la revendication 6, dans lequel le premier port (32) au niveau d'un second circuit de l'échangeur de chaleur (10) est relié à la sortie (22) de l'évaporateur (16).

8. Dispositif selon la revendication 6 ou 7, dans lequel l'échangeur de chaleur (10) fait partie de la chambre de mesure (12), le second port au niveau du second circuit de l'échangeur de chaleur (10) formant de préférence une sortie (34) de la chambre de mesure (12).

9. Dispositif selon la revendication 8, dans lequel le capteur de température et le capteur d'humidité sont disposés comme capteur mixte (38) au niveau d'une bride à la sortie (34) de la chambre de mesure (12).

10. Dispositif selon l'une des revendications 1 à 9, dans lequel un circuit de commande (40) est prévu, lequel circuit de commande est relié au capteur de température et au capteur d'humidité pour détecter les valeurs réelles par l'intermédiaire de première et deuxième lignes de données (42 ; 44).

11. Dispositif selon l'une des revendications 10, dans lequel une valeur réelle de la température peut être fournie au circuit de commande (40) dans la chambre d'incubation (50), de préférence par l'intermédiaire d'une troisième ligne de données (54).

12. Dispositif selon la revendication 10, dans lequel la valeur de consigne de l'humidité relative au niveau d'une unité d'entrée (56) peut être entrée et transmise au circuit de commande (40) par l'intermédiaire d'une quatrième ligne de données (58).

13. Dispositif selon l'une des revendications 11 ou 12, dans lequel le circuit de commande (40) calcule une masse d'eau absolue à partir des valeurs réelles, à partir de laquelle une valeur réelle d'humidité relative dans la chambre d'incubation (50) peut être déduite.

14. Dispositif selon l'une des revendications 1 à 13, dans lequel une micropompe (28) pouvant être dosée est prévue, laquelle micropompe permet de régler le réservoir d'eau (24).

15. Dispositif selon l'une des revendications 1 à 14, dans lequel le tuyau (46) et/ou le réservoir (24) sont conçus de telle manière que chacun d'eux puisse être stérilisé.
